(19) Europäisches Patentamt
European Patent Office
Office européen des brevets

(11) Publication number: 0 174 780 A2

# (12) EUROPEAN PATENT APPLICATION

(21) Application number: 85306151.3

(51) Int. Cl.[4]: C01B 33/28

(22) Date of filing: 30.08.85

(30) Priority: 05.09.84 US 647506
17.09.84 US 651149

(43) Date of publication of application:
19.03.86 Bulletin 86/12

(84) Designated Contracting States:
BE DE FR GB IT NL SE

(71) Applicant: MOBIL OIL CORPORATION
150 East 42nd Street
New York New York 10017(US)

(72) Inventor: Chu, Pochen
1173 Ollerton Road
West Deptford New Jersey 08066(US)
Inventor: Herbst, Joseph Anthony
60 Bryant Road
Turnersville New Jersey 08012(US)
Inventor: Klocke, Donald Joseph
209 Roberts Drive
Somerdale New Jersey 08081(US)
Inventor: Rubin, Mae Koenig
50 Belmont Avenue
Bala Cynwyd Pennsylvania 19004(US)
Inventor: Vartuli, James Clarke
320 Ponds Edge Road
West Chester Pennsylvania 19380(US)

(74) Representative: West, Alan Harry
Mobil Court 3 Clements Inn
London WC2A 2EB(GB)

## (54) A crystalline metallosilicate, the synthesis thereof and its use in organic conversion.

(57) There is disclosed an alumino- or borosilicate zeolite having an $SiO_2$ to $Al_2O_3$ or $B_2O_3$ molar ratio of 5-500 and the x-ray diffraction pattern shown in Table I of the specification. The zeolite, which is useful as a catalyst in organic conversions, can be produced from an aqueous reaction mixture containing a 2-(hydroxyalkyl)trialkylammonium cation.

EP 0 174 780 A2

# A CRYSTALLINE METALLOSILICATE, THE SYNTHESIS THEREOF AND ITS USE IN ORGANIC CONVERSION

This invention relates to a crystalline metallosilicate, the synthesis thereof and its use in organic conversion.

Zeolitic materials, both natural and synthetic, have been demonstrated to have catalytic properties for various types of hydrocarbon conversion. Certain zeolitic materials are ordered, porous crystalline aluminosilicates having a definite crystalline structure as determined by X-ray diffraction, within which there are a large number of small cavities which are interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline aluminosilicates. These aluminosilicates can be described as a rigid three-dimensional framework of $SiO_4$ and $AlO_4$ in which the tetrahedra are cross-linked by the sharing of oxygen atoms whereby the ratio of the total aluminum and silicon atoms to oxygen atoms is 1:2. The electrovalence of the tetrahedra containing aluminum is balanced by the inclusion in the crystal of a cation, for example an alkali metal or an alkaline earth metal cation. This can be expressed wherein the ratio of aluminum to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given aluminosilicate by suitable selection of the cation. The spaces between the tetrahedra are occupied by molecules of water prior to dehydration.

Prior art techniques have resulted in the formation of a great variety of synthetic zeolites. The zeolites have come to be designated by letter or other convenient symbols, as illustrated by zeolite A (U. S. Patent 2,882,243), zeolite X (U. S. Patent 2,882,244), zeolite Y (U. S. Patent 3,130,007), zeolite ZK-5 (U.S. Patent 3,247,195), zeolite ZK-4 (U. S. Patent 3,314,752), zeolite ZSM-5 (U. S. Patent 3,702,886), zeolite ZSM-11 (U. S. Patent 3,709,979), zeolite ZSM-12 (U. S. Patent 3,832,449), zeolite ZSM-20 (U. S. Patent 3,972,983), ZSM-35 (U. S. Patent 4,016,245), ZSM-38 (U. S. Patent 4,046,859), and zeolite ZSM-23 (U. S. Patent 4,076,842).

The $SiO_2/Al_2O_3$ mole ratio of a given aluminosilicate zeolite is often variable. For example, zeolite X can be synthesized with $SiO_2/Al_2O_3$ ratios of from 2 to 3; zeolite Y, from 3 to about 6. In some zeolites, the upper limit of the $SiO_2/Al_2O_3$ ratio is unbounded. ZSM-5 is one such example wherein the $SiO_2/Al_2O_3$ ratio is at least 5 and up to infinity. U. S. Patent 3,941,871 (Re. 29,948) discloses a porous crystalline silicate made from a reaction mixture containing no deliberately added alumina and exhibiting the X-ray diffraction pattern characteristic of ZSM-5 type zeolites. U. S. Patents 4,061,724, 4,073,865 and 4,104,294 describe crystalline silicates or organosilicates of varying alumina and metal content.

A number of synthetic zeolites have been prepared which may be said to be isostructural with naturally occurring zeolites. Zeolites ZSM-35 and ZSM-38 are, for instance, ferrierite-type zeolites. Zeolite ZK-20 (U.S. Patent 3,459,676) is described as being isostructural with the naturally occurring zeolite levynite.

Although the term "zeolites" has sometimes been used to define materials containing silica and alumina, it is recognized that the silica and alumina portions may be replaced in whole or in part with other oxides. More particularly, $GeO_2$ is an art recognized substitute for $SiO_2$ and $B_2O_3$ is an art recognized replacement for $Al_2O_3$. Accordingly, the term zeolite is used herein to connote not only materials containing silicon and, optionally, aluminum atoms in the crystalline lattice structure thereof, but also materials which contain suitable replacement atoms for such silicon and/or aluminum.

According to one aspect of the invention, there is provided a synthetic porous crystalline metallosilicate zeolite having a silica to metal oxide molar ratio of from 5 to 500, wherein said metal is aluminum or boron and said porous crystalline material in the uncalcined state is characterized by an x-ray diffraction pattern having values substantially as set forth in Table 1 of the specification.

According to another aspect of the invention, there is provided a method for preparing the above-mentioned synthetic porous crystalline metallosilicate zeolite which comprises preparing a mixture containing sources of alkali metal ions, an oxide of aluminum or boron, an oxide of silicon, a 2-(hydroxyalkyl)trialkylammonium cation and water and maintaining said reaction mixture under conditions such that said crystalline material is formed.

According to yet another aspect of the invention, there is provided a process for effecting catalytic conversion of an organic charge which comprises contacting said charge under catalytic conversion conditions with a catalyst comprising a synthetic porous crystalline metallosilicate zeolite material according to said one aspect of the invention.

The metallosilicate zeolite of the present invention may be considered to be a aluminum- or boron-containing counterpart of the substantially aluminum free zeolites described in U.S. Patent No. 4,376,757.

In its as synthesized form, the metallosilicate zeolite of the present invention may have at least 0.5, more preferably at least 0.8, moles of 2-(hydroxyalkyl)-trialkylammonium directing agent per mole of aluminum. The term "directing agent", as used herein, connotes a compound which is added to the crystallization mixture used to form the zeolite in order to influence the formation of the ultimately formed crystal lattice. At least a portion of the cations corresponding to the directing agent are bound to anionic sites of the crystal lattice in the as synthesized form of the zeolite.

The catalytically active metallosilicate zeolite described herein, prior to calcination, has an X-ray diffraction pattern which essentially corresponds to Table I as set forth in U.S. Patent No. 4,376,757. This Table I sets forth the following observable major peaks:

TABLE I

| Interplanar Spacing d(A) Observed | Relative Intensity |
|---|---|
| 10.90 | VS |
| 7.02 | W |
| 5.09 | W |
| 4.32 | MS |
| 4.17 | W |
| 3.97 | M |
| 3.66 | M |
| 3.53 | VS |
| 3.35 | MS |
| 3.32 | MS |
| 3.23 | M |
| 2.88 | W |
| 2.78 | VW |
| 2.62 | W |
| 1.97 | W |
| 1.88 | W |
| 1.85 | W |

These values were determined by standard X-ray diffraction powder techniques. The radiation was the K-alpha doublet of copper, and a scintillation counter spectrometer with a strip chart pen recorder was used. The peak heights, I, and the positions as a function of 2 , where is the Bragg angle, were read from the spectrometer chart. From these, the relative intensities 100 $I/I_o$, where $I_o$is the intensity of the strongest line or peak, and d(obs.), the interplanar spacing in A corresponding to the recorded lines, were calcuated. In Table I the relative intensities are given in terms of the symbols as follows: VW = very weak (less than 10), W = weak (10-19), M = medium (20-39), MS = medium strong (40-70) and VS = very strong (greater than 70). Ion exchange of the original cations with other cations reveals substantially the same pattern with some minor shifts in interplanar spacing and variation in relative intensity. Other minor variations can occur depending on the metal to silicon ratio of the particular sample and on whether it had been subjected to thermal treatment.

A major change in the X-ray diffraction pattern is, however, observed when the as synthesized form of the aluminosilicate zeolite is calcined to remove the organic directing agent. Such calcination may involve heating at temperatures of about 538°C (1000°F) for about 16 hours. More particularly, Table II shows X-ray diffraction data for an as synthesized form of an aluminosilicate zeolite in accordance with the present invention.

TABLE II

| LINE NUMBER | 2THETA | D(A) | I/IMAX |
|---|---|---|---|
| 1 | 8.11 | 10.91 | 100 |
| 2 | 12.58 | 7.04 | 4 |
| 3 | 13.48 | 6.57 | 2 |
| 4 | 14.07 | 6.29 | 3 |
| 5 | 14.40 | 6.15 | 1 |
| 6 | 14.80 | 5.99 | 1 |
| 7 | 16.26 | 5.45 | 1 |
| 8 | 16.98 | 5.22 | 2 |
| 9 | 17.09 | 5.19 | 2 |
| 10 | 17.46 | 5.08 | 9 |
| 11 | 17.84 | 4.97 | 2 |
| 12 | 18.21 | 4.87 | 1 |
| 13 | 20.63 | 4.31 | 19 |
| 14 | 21.24 | 4.18 | 4 |
| 15 | 22.13 | 4.02 | 1 |
| 16 | 22.44 | 3.96 | 3 |
| 17 | 22.81 | 3.90 | 2 |
| 18 | 23.92 | 3.72 | 3 |
| 19 | 24.33 | 3.66 | 9 |
| 20 | 24.51 | 3.63 | 8 |
| 21 | 25.11 | 3.55 | 17 |
| 22 | 25.34 | 3.51 | 20 |
| 23 | 26.69 | 3.34 | 7 |
| 24 | 26.95 | 3.31 | 12 |
| 25 | 27.70 | 3.22 | 7 |
| 26 | 31.03 | 2.882 | 1 |
| 27 | 32.29 | 2.772 | 1 |
| 28 | 34.22 | 2.621 | 1 |
| 29 | 35.44 | 2.533 | 1 |
| 30 | 35.96 | 2.498 | 1 |
| 31 | 38.28 | 2.351 | 1 |
| 32 | 42.08 | 2.147 | 1 |
| 33 | 44.73 | 2.026 | 1 |
| 34 | 46.04 | 1.971 | 3 |
| 35 | 48.32 | 1.884 | 1 |
| 36 | 49.13 | 1.854 | 1 |
| 37 | 51.73 | 1.767 | 1 |
| 38 | 56.25 | 1.635 | 1 |

However, after calcination and conversion to the hydrogen form, the aluminosilicate zeolite shows the x-ray diffraction data as set forth in Table III.

TABLE III

| LINE NUMBER | 2THETA | D(A) | I/IMAX |
|---|---|---|---|
| 1 | 9.49 | 9.32 | 100 |
| 2 | 12.80 | 6.92 | 21 |
| 3 | 12.86 | 6.88 | 26 |
| 4 | 13.51 | 6.55 | 2 |
| 5 | 14.35 | 6.17 | 4 |
| 6 | 15.88 | 5.58 | 2 |
| 7 | 18.13 | 4.89 | 1 |
| 8 | 19.13 | 4.64 | 3 |
| 9 | 19.77 | 4.49 | 7 |
| 10 | 20.21 | 4.39 | 6 |
| 11 | 21.41 | 4.15 | 5 |
| 12 | 22.70 | 3.92 | 8 |
| 13 | 23.12 | 3.85 | 8 |
| 14 | 24.10 | 3.69 | 6 |
| 15 | 25.63 | 3.48 | 13 |
| 16 | 25.94 | 3.44 | 17 |
| 17 | 26.75 | 3.33 | 12 |
| 18 | 27.33 | 3.26 | 6 |
| 19 | 27.70 | 3.22 | 2 |
| 20 | 28.82 | 3.10 | 2 |
| 21 | 29.83 | 2.996 | 1 |
| 22 | 31.62 | 2.830 | 1 |
| 23 | 32.40 | 2.763 | 1 |
| 24 | 34.80 | 2.578 | 1 |
| 25 | 49.47 | 1.842 | 1 |

Similarly, a major change in the x-ray diffraction pattern is observed when the as synthesized form of the borosilicate zeolite is calcined to remove the organic directing agent. More particularly, Table IV shows x-ray diffraction data for an as synthesized form of a borosilicate zeolite in accordance with the present invention.

TABLE IV

| LINE NUMBER | 2THETA | D(A) | I/IMAX |
|---|---|---|---|
| 1 | 8.16 | 10.84 | 100 |
| 2 | 12.64 | 7.00 | 14 |
| 3 | 13.42 | 6.60 | 5 |
| 4 | 13.60 | 6.51 | 6 |
| 5 | 13.90 | 6.37 | 4 |
| 6 | 14.19 | 6.24 | 4 |
| 7 | 14.38 | 6.16 | 3 |
| 8 | 17.58 | 5.05 | 15 |
| 9 | 18.44 | 4.81 | 2 |
| 10 | 20.75 | 4.28 | 45 |
| 11 | 22.57 | 3.94 | 14 |
| 12 | 22.83 | 3.90 | 7 |
| 13 | 24.37 | 3.65 | 22 |
| 14 | 24.68 | 3.61 | 9 |
| 15 | 25.22 | 3.53 | 51 |
| 16 | 25.45 | 3.50 | 54 |
| 17 | 26.82 | 3.32 | 20 |
| 18 | 27.05 | 3.30 | 33 |
| 19 | 27.81 | 3.21 | 21 |
| 20 | 28.88 | 3.09 | 1 |
| 21 | 30.44 | 2.937 | 1 |
| 22 | 31.11 | 2.875 | 4 |
| 23 | 32.41 | 2.762 | 2 |
| 24 | 34.33 | 2.612 | 4 |
| 25 | 35.55 | 2.525 | 1 |
| 26 | 36.13 | 2.486 | 2 |
| 27 | 37.50 | 2.398 | 1 |
| 28 | 38.47 | 2.340 | 1 |
| 29 | 40.60 | 2.222 | 1 |
| 30 | 42.21 | 2.141 | 3 |
| 31 | 45.00 | 2.015 | 1 |
| 32 | 46.24 | 1.963 | 6 |
| 33 | 46.75 | 1.943 | 1 |
| 34 | 47.19 | 1.926 | 2 |
| 35 | 48.56 | 1.875 | 4 |
| 36 | 49.19 | 1.852 | 4 |
| 37 | 49.96 | 1.825 | 1 |
| 38 | 52.27 | 1.750 | 1 |
| 39 | 56.12 | 1.639 | 1 |
| 40 | 56.60 | 1.626 | 1 |

After calcination and conversion to the hydrogen form, the borosilicate zeolite exhibits the x-ray diffraction data set out in Table V.

TABLE V

| LINE NUMBER | 2THETA | D(A) | I/IMAX |
|---|---|---|---|
| 1 | 9.73 | 9.09 | 100 |
| 2 | 12.86 | 6.88 | 74 |
| 3 | 14.38 | 6.16 | 9 |
| 4 | 16.10 | 5.51 | 7 |
| 5 | 18.25 | 4.86 | 3 |
| 6 | 19.20 | 4.62 | 5 |
| 7 | 19.97 | 4.45 | 12 |
| 8 | 20.54 | 4.32 | 4 |
| 9 | 21.62 | 4.11 | 13 |
| 10 | 22.91 | 3.88 | 16 |
| 11 | 23.23 | 3.83 | 16 |
| 12 | 24.10 | 3.69 | 10 |
| 13 | 25.88 | 3.44 | 42 |
| 14 | 26.85 | 3.32 | 16 |
| 15 | 27.54 | 3.24 | 11 |
| 16 | 28.89 | 3.09 | 1 |
| 17 | 36.90 | 2.436 | 1 |
| 18 | 44.30 | 2.045 | 1 |
| 19 | 46.50 | 1.953 | 1 |
| 20 | 49.38 | 1.846 | 2 |
| 21 | 50.42 | 1.810 | 1 |

The metallosilicate zeolite of the present invention sorbs significant amounts of commonly used test adsorbate materials, i.e. cyclohexane, n-hexane and water. For example, an aluminosilicate zeolite of the present invention has been observed to sorb 1.6 weight percent cyclohexane and 1.2 weight percent n-hexane, wherein cyclohexane and n-hexane sorption are measured at 20 Torr.

The zeolite of the present invention can be used either in the alkali metal form, e.g. the sodium or potassium form; the ammonium form; the hydrogen form or another univalent or multivalent cationic form. When used as a catalyst the zeolite may be subjected to thermal treatment to remove part or all of the organic constituent.

The original alkali metal cations of the as synthesized zeolite can be replaced in accordance with techniques well known in the art, at least in part, by ion exchange with other cations. Preferred replacing cations include metal ions, hydrogen ions, hydrogen precursor, e.g. ammonium, ions and mixtures thereof. Particularly preferred cations are those which render the zeolite catalytically active, especially for hydrocarbon conversion. Replacing cations include hydrogen, rare earth metals and metals of Groups IA, IIA, IIIA, IVA, IB, IIB, IIIB, IVB and VIII of the Periodic Table of the Elements.

A typical ion exchange technique would be to contact the synthetic metallosilicate zeolite with a salt of the desired replacing cation or cations. Examples of such salts include the halides, e.g. chlorides, nitrates and sulfates.

For example, the zeolite can be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition to the extent atom, aluminum, is in the structure, impregnated therein or intimately physically admixed therewith. Such component can be impregnated in or on to it such as for example, by, in the case of platinum, treating the zeolite with a solution containing a platinum metal-containing ion. Thus, suitable platinum compounds include chloroplatinic acid, platinous chloride and various compounds containing the platinum amine complex.

The zeolite, especially in its metal, hydrogen and ammonium forms can be beneficially converted to another form by thermal treatment. This thermal treatment is generally performed by heating one of these forms at a temperature of at least 370°C for at least 1 minute and generally not longer than 20 hours. While subatmospheric pressure can be employed for the thermal treatment, atmospheric pressure is desired for reasons of convenience. The thermal treatment can be performed at a temperature up to about 925°C.

The new zeolite, when employed either as an adsorbent or as a catalyst in an organic compound conversion process should be dehydrated, at least partially. This can be done by heating to a temperature in the range of 200°C to 595°C in an inert atmosphere, such as air, nitrogen, etc. and at atmospheric, subatmospheric or superatmospheric pressures for between 30 minutes and 48 hours. Dehydration can also be performed at room temperature merely by placing the aluminosilicate zeolite in a vacuum, but a longer time is required to obtain a sufficient amount of dehydration.

The zeolite can be prepared from a reaction mixture containing sources of alkali metal ions (Z), an oxide of aluminum or boron, an oxide of silica, water and an organic cation (R), wherein R is a 2-(hydroxyalkyl)trialkylammonium cation wherein alkyl is composed of of one or two carbon atoms. The reaction mixture may comprise an appropriate selection of reactants, having a composition, in terms of mole ratios, falling within the following ranges:

| Reactants | Useful | Preferred |
|---|---|---|
| $SiO_2/Al_2O_3$ or $B_2O_3$ | 10 - 1000 | 20 - 250 |
| $OH^-/SiO_2$ | 0.1 - 0.8 | 0.2 - 0.6 |
| $H_2O/OH^-$ | 20 - 100 | 20 - 60 |
| R/(R+Z) | 0.1 - 0.8 | 0.2 - 0.6 |
| K/(K+Na) | 0.2 - 1.0 | 0.2 - 0.7 |

wherein R and Z are as above defined.

The alumina sources useful herein exhibit a limited degree and rate of solubility in either water or caustic solution. This is conveniently measured as "degree of solubility", which is the weight percent of aluminum ions provided to the subject solution at particular conditions of temperature and time by the subject source of alumina. Degree of solubility of said alumina source is preferably less than about 20 weight percent, preferably less than about 5 weight percent, in water solution determined by the following solubility test:

Five grams of alumina source (100% solid basis) are mixed and slurried with 100 grams of distilled water. The slurried mixture is heated to 100°C and maintained at this temperature for 24 hours. The mixture is then filtered while at about 100°C. Degree of solubility is then determined by analyzing the filtrate for aluminium ion content and drying and weighing the filtered material.

Degree of solubility of said alumina source is preferably less than about 90 weight percent, preferably less than about 50 weight percent, in caustic solution determined by the following solubility test:

Five grams of alumina source (100% solid basis) are mixed and slurried with 100 grams of 5 weight percent sodium hydroxide solution (5 grams of anhydrous sodium hydroxide dissolved in 95 grams of distilled water). The slurried mixture is heated to 100°C and maintained at this temperature for 24 hours. The mixture is then filtered while at about 100°C. Degree of solubility is then determined by analyzing the filtrate for aluminum ion content and drying and weighing the filtered material.

For either caustic or water solution,

$$\text{Degree of Solubility} = \frac{\left(5 - \text{Remaining Undissolved Alumina, grams}\right)}{5} \times 100.$$

Commonly used sources of alumina for zeolite synthesis, such as, for example, sodium aluminate or aluminum sulfate, exhibit degrees of solubility by either of the above solubility tests of 100 weight percent. In fact, both sodium aluminate and aluminum sulfate are essentially instantaneously 100 weight percent soluble in distilled water at room temperature.

Alumina sources found to satisfy the above solubility limitations include the high temperature transition aluminas, such as, for example, kappa, theta, iota and the two delta forms of alumina; transition aluminas, such as, for example, gamma, eta and chi forms of alumina; and the tri- and monohydrated aluminas which are not predigested or reacted with alkaline earth or alkali metal hydroxide, strong mineral acids, e.g. hydrogen fluoride. Such hydrated aluminas useful herein include the trihydrates known as gibbsite, bayerite and nordstrandite, and the monohydrates known as boehmite and diaspore.

The boron source useful in making the borosilicate of the present invention is preferably boric acid which may be considered as a hydrated form of $B_2O_3$, particularly in view of the equation:

$$B_2O_3 + 3H_2O = 2H_3BO_3.$$

Crystallization of the new aluminosilicate zeolite can be carried out at either static or stirred conditions in a suitable reactor vessel, such as for example, polypropylene jars or teflon lined or stainless steel autoclaves. A useful range of temperatures for crystallization is from 60°C to 350°C for a time of 12 hours to 200 days. The pH may be from 7 to 14.0. Thereafter, the crystals are separated from the liquid and recovered. The composition can be prepared utilizing materials which supply the appropriate oxides. Such compositions may include sodium silicate, silica hydrosol, silica gel, silicic acid, sodium hydroxide, a source of aluminum, and an appropriate organic compound. It should be realized that the reaction mixture component oxides can be supplied from more than one source. The reaction mixture can be prepared either batchwise or continuously. Crystal size and crystallization time of the new crystalline aluminosilicate zeolite will vary with the nature of the reaction mixture employed and the crystallization conditions.

In all cases, synthesis of the present metallosilicate crystals is facilitated by the presence of at least 0.01 percent, preferably 0.10 percent and still more preferably 1 percent, seed crystals (based on total weight) of crystalline product.

The 2-(hydroxyalkyl)trialkylammonium directing agent may be the hydroxide or halide, e.g. chloride, iodide or bromide. Preferably, the compound is 2-(hydroxyethyl)trimethylammonium chloride, normally called choline chloride.

The crystals prepared by the instant invention can be shaped into a wide variety of particle forms. Generally speaking, the particles can be in the form of a powder, a granule, or a molded product, such as an extrudate having particle size sufficient to pass through a 2 mesh (Tyler) screen and be retained on a 400 mesh (Tyler) screen. In cases where the catalyst is molded, such as by extrusion, the crystals can be extruded before drying or partially dried and then extruded.

In the case of many catalysts, it is desired to incorporate the new metallosilicate zeolite with another material resistant to the temperatures and other conditions employed in organic conversion processes. Such materials include active and inactive material and synthetic or naturally occurring zeolites as well as inorganic materials such as clays, silica and/or metal oxides, e.g. alumina. The latter may be either naturally occurring or in the form of gelatinous precipitates or gels including mixtures of silica and metal oxides. Use of a material in conjunction with the new zeolite crystal, i.e. combined therewith, which is active, tends to improve the conversion and/or selectivity of the catalyst in certain organic conversion processes. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc., function as binders for the catalyst. It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the new crystal include the montmorillonite and kaolin families which include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification.Binders useful for compositing with the present crystal also include inorganic oxides, notably alumina.

In addition to the foregoing materials, the zeolite can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. The relative proportions of finely divided crystalline material and inorganic oxide gel matrix vary widely, with the zeolite content ranging from 1 to 90 percent by weight and more usually, particularly when the composite is prepared in the form of beads, in the range of 2 to 80 weight percent of the composite.

The metallosilicate zeolite of the present invention is useful as catalyst component for a variety of organic, e.g. hydrocarbon, compound conversion processes. Such conversion processes include, as non-limiting examples, cracking hydrocarbons with reaction conditions including a temperature of from 300°C to 700°C, a pressure of from 10 to 3040 kPa (0.1 to 30 atmospheres) and a weight hourly space velocity of from 0.1 to 20; dehydrogenating hydrocarbon compounds with reaction conditions including a temperature of from 300°C to 700°C, a pressure of from 10 to 1013 kPa (0.1 to 10 atmospheres) and a weight hourly space velocity of from 0.1 to 20; converting paraffins to aromatics with reaction conditions including a temperature of from 100°C to 700°C, a pressure of from 10 to 6080 kPa (0.1 to 60 atmospheres), a weight hourly space velocity of from 0.5 to 400 and a hydrogen/hydrocarbon mole ratio of from 0 to 20; converting olefins to aromatics, e.g. benzene, toluene and xylenes, with reaction conditions including a temperature of from 100°C to 700°C, a pressure of from 10 to 6080 kPa (0.1 to 60 atmospheres), a weight hourly space velocity of from 0.5 to 400 and a hydrogen/hydrocarbon mole ratio of from 0 to 20; converting alcohols, e.g. methanol, or ethers, e.g. dimethylether, or mixtures thereof to hydrocarbons including aromatics with reaction conditions including a temperature of from 275°C to 600°C, a pressure of from 51 to 5066 kPa (0.5 to 50 atmospheres) and a liquid hourly space velocity of from 0.5 to 100; isomerizing xylene feedstock components with reaction conditions including a temperature of from 230°C to 510°C, a pressure of from 304 to 3546 kPa (3 to 35 atmospheres), a weight hourly space velocity of from 0.1 to 200 and a hydrogen/hydrocarbon mole ratio of from 0 to 100; disproportionating toluene with reaction conditions including a temperature of from 200°C to 760°C, a pressure of from 101 to 6080 kPa (1 to 60 atmospheres) and a weight hourly space velocity of from 0.08 to 20; alkylating aromatic hydrocarbons, e.g. benzene and alkylbenzenes, in the presence of an alkylating agent, e.g. olefins, formaldehyde, alkyl halides and alcohols, with reaction conditions including a temperature of from 340°C to 500°C, a pressure of from 101 to 20265 kPa (1 to 200 atmospheres), a weight hourly space velocity of from 2 to 2000 and an aromatic hydrocarbon/alkylating agent mole ratio of from 1/1 to 20/1; and transalkylating aromatic hydrocarbons in the presence of polyalkylaromatic hydrocarbons with reaction conditions including a temperature of from 340°C to 500°C, a pressure of from 101 to 20265 kPa (1 to 200 atmospheres), a weight hourly space velocity of from 10 to 1000 and an aromatic hydrocarbon/polyalkylaromatic hydrocarbon mole ratio of from 1/1 to 16/1.

When Alpha value is examined in the Examples which follow, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of the highly active silica-alumina cracking catalyst taken as an Alpha Value of 1 (Rate Constant = 0.016 $sec^{-1}$). The Alpha Test is described in U.S. Patent 3,354,078 and in The Journal of Catalysis, Vol, IV, pp. 522-529 (August 1965).

## EXAMPLE 1

A number of individual alumina sources were tested for their degree of solubility by the above-defined tests for both water and caustic solution. The tested sources were:

A. Primarily spray-dried and milled transition alumina with traces of alpha monodydrate alumina.

B. Spray dried alpha monohydrate alumina.

C. Dried alpha monohydrate alumina.

D. Rotary kiln dried alpha monodydrate alumina.

E. Beta trihydrate and alpha monohydrate aluminas in the form of wet filter cake.

F. Primarily transition alumina with traces of beta trihydrate and alpha monohydrate alumina.

G. Dried alpha monohydrate alumina.

Five grams of each source were individually mixed and slurried with 100 grams of distilled water, heated to 100°C and maintained in a slurried condition at 100°C for 24 hours. Also, five grams of each source were individually mixed and slurried with 100 grams of 5 weight percent sodium hydroxide solution (prepared by dissolving 5 grams of anhydrous sodium hydroxide in 95 grams of distilled water), heated to 100°C and maintained in a slurried condition at 100°C for 24 hours. The mixtures were then filtered at 100°C and the filtrates were analyzed for aluminum ion content. The resulting filter cakes were dried and weighed. Results of this testing were as listed below:

| | Degree of Solubility, Wt. % | |
|---|---|---|
| Alumina Source | Water Solution | Caustic Solution |
| A | - | 40.4 |
| B | less than 0.2 | 31.4 |
| C | less than 0.2 | 39.8 |
| D | less than 0.2 | 28.4 |
| E | less than 0.2 | 31.2 |
| F | less than 0.2 | 45.8 |
| G | less than 0.2 | 47.2 |
| H | 4.2 | 49.2 |

In the following Examples 2-7, the primarily transition alumina with traces of beta trihydrate and alpha monohydrate, designated in Example 1 as alumina source F, was used as the alumina source. The following were used as silica sources:

A. An aqueous solution of colloidal silica (30 percent by weight).

B. A high surface area amorphous silica powder.

EXAMPLE 2

A solution was prepared by mixing one part (by weight) alumina, 14 parts sodium hydroxide (98% by weight), 33 parts potassium hydroxide (86% by weight) and 263 parts water. To this solution 111 parts choline chloride, 380 parts silica source A, and 28 parts ZSM-34 zeolite material were added and the mixture was stirred for approximately 15 minutes. The total mixture was then poured into teflon bottles and placed in a steam autoclave at 149°C (300°F) for seven days. The resultant crystalline material was then filtered and washed on a Buchner funnel and then dried overnight at 121°C (250°F). The analysis indicated the aluminosilicate zeolite of the present invention having a silica to alumina molar ratio of 73.

EXAMPLE 3

A solution was prepared by mixing one part (by weight) alumina, 7 parts sodium hydroxide (98% by weight), 15 parts potassium hydroxide (86% by weight) and 128 parts water. To this solution 54 parts choline chloride and 185 parts silica source A were added and the mixture was stirred for approximately 15 minutes. The total mixture was poured into a stirred autoclave. The autoclave was heated to 160°C (320°F) with constant stirring and maintained for 72 hours. The resultant crystalline material was then filtered and washed on a Buchner funnel and then dried overnight at 121°C (250°F). The analysis indicated the aluminosilicate zeolite of the present invention having a silica to alumina ratio of 130.

EXAMPLE 4

A solution was prepared by mixing one part (by weight) alumina, 3.5 parts sodium hydroxide (98% by weight), 8 parts potassium hydroxide (86% by weight) and 64 parts water. To this solution 27 parts choline chloride and 93 parts silica source A were added and the mixture was stirred for approximately 15 minutes. The total mixture was poured into a stirred autoclave. The autoclave was heated to 160°C (320°F) with constant stirring and maintained for 72 hours. The resultant crystalline material was then filtered and washed on a Buchner funnel and then dried overnight at 121°C (250°F). The analysis indicated the aluminosilicate zeolite of the present invention having a silica to alumina molar ratio of 40.

EXAMPLE 5

A solution was prepared by mixing one part (by weight) alumina, 7 parts sodium hydroxide (98% by weight), 6 parts potassium hydroxide (86% by weight) and 128 parts water. This solution was added to a stirred autoclave. Another solution was prepared by mixing 54 parts choline chloride, 185 parts silica source A and 1.4 parts ZSM-34 zeolite material and then this mixture was added to the autoclave. The total mixture was stirred at room temperature for approximately 15 minutes. The autoclave was heated to 143°C (290°F) with constant stirring and maintained for 7 days. The resultant crystalline material was then filtered and washed on a Buchner funnel and then dried overnight at 121°C (250°F). The analysis indicated the aluminosilicate zeolite of the present invention having a silica to alumina molar ratio of 78.

EXAMPLE 6

A solution was prepared by mixing one part (by weight) alumina, 7 parts sodium hydroxide (98% by weight), 15 parts potassium hydroxide (86% by weight) and 209 parts water. This solution was added to a stirred autoclave. Another solution was prepared by mixing 54 parts choline chloride and 130 parts silica source B and then was added to the autoclave. The total mixture was stirred at room temperature for approximately 15 minutes. The autoclave was heated to 160°C (320°F) with constant stirring and maintained for 72 hours. The resultant crystalline material was then filtered and washed on a Buchner funnel and then dried overnight at 121°C (250°F). The analysis indicated the aluminosilicate zeolite of the present invention having a silica to alumina ratio of 106.

EXAMPLE 7

The zeolite from Example 5 was mixed with gamma alumina to make a mixture of 65 parts (by weight) zeolite and 35 parts alumina. Enough water was added to the mixture so that the resulting catalyst could be formed into 1.6 mm (1/16") extrudates. These extrudates were activated by first, calcining in nitrogen at 538°C (1000°F) followed by aqueous exchanges with an ammonium nitrate solution and finally calcining in air at 538°C (1000°F). The alpha value at 528°C was 4.

The catalyst was loaded into a straight tube (downflow) reactor [1.9 cm (3/4" I.D.)] and heated to 357°C (675°F) in flowing nitrogen. A reaction mixture comprising of 50/50 (by weight) methanol/water was passed over the catalyst at a WHSV of 1 (methanol) at 101 kPa (0 psig). The products were analyzed and the results are as follows:

| | |
|---|---|
| Methanol Conversion | 3% |
| Selectivity (weight of hydrocarbon) | |
| Methane | 6.5 |
| $C_2^=$ | 45.7 |
| $C_3^=$ | 7.3 |
| $C_4^=$ | 14.7 |
| $C_2$-$C_4$ Paraffins | 9.5 |

EXAMPLE 8

A solution of 104.0 grams of choline chloride, 3.2 grams of $H_3BO_3$. 12.0 grams NaOH and 400 grams of water was slowly added to 160.0 grams of colloidal silica (30% $SiO_2$). The mixture was placed in a teflon jar and crystallized at 150°C. A sample removed after 9 days was subjected to x-ray analysis and was characterized as the borosilicate zeolite of the present invention. After additional crystallization of 5 days at 150°C, the material was filtered, water washed and dried at 120°C. This material had about 90 percent of the purity of the earlier analyzed 9 day sample. The dried solid had the following composition:

| | |
|---|---|
| $SiO_2$. wt% | 78.0 |
| $B_2O_3$ | 0.60 |
| $Na_2O$ | 0.20 |
| $Al_2O_3$ | 0.057 |
| N | 2.14 |
| Ash | 81.0 |
| Molar Ratios $SiO_2/B_2O_3$ | 140 |
| $SiO_2/Al_2O_3$ | 2346 |

EXAMPLES 9 AND 10

Two additional samples of the borosilicate zeolite of the present invention were prepared. Reaction conditions and results are summarized in Table VI.

TABLE VI

| Example No. | 2 | 3 |
|---|---|---|
| Reaction Mixture, g | | |
| A. Choline Chloride | 104.0 | 200.0 |
| $H_3BO_3$ | 3.2 | 6.4 |
| NaOH | 12.0 | 24.0 |
| $H_2O$ | 400.0 | 800.0 |
| B. Colloidal $SiO_2$ (30%) | 160.0 | 320.0 |
| Crystallization | Teflon jar | Stirred |
| Temp. °C | 150 | 145 |
| Time, days | 14 | 13 |
| Composition, wt% | | |
| $SiO_2$ | 79.9 | 79.1 |
| $B_2O_3$ | 0.54 | 0.60 |
| $Na_2O$ | 0.22 | 0.32 |
| $Al_2O_3$ | 0.07 | 0.09 |
| N | 2.06 | --.-- |
| Ash | 81.4 | 80.9 |
| Molar Ratios | | |
| $SiO_2/B_2O_3$ | 151 | 142 |
| $SiO_2/Al_2O_3$ | 1939 | 1494 |

**Claims**

1. A synthetic porous crystalline metallosilicate zeolite having a silica to metal oxide molar ratio of from 5 to 500, wherein said metal is boron or aluminum and said porous crystalline material in the uncalcined state characterized by an x-ray diffraction pattern having values substantially as set forth in Table 1 of the specification.

2. The crystalline material of Claim 1 wherein said metal is aluminum.

3. The crystalline material of claim 1 wherein said metal is boron.

4. A method for preparing the synthetic porous crystalline material of Claim 1 which comprises preparing a mixture containing sufficient sources of alkali metal ions, an oxide of boron or aluminum, an oxide of silicon, a 2-(hydroxyalkyl)trialkylammonium cation and water, and maintaining said reaction mixture under sufficient conditions until said crystalline material is formed.

5. The method of Claim 4 wherein said mixture has the following composition, in terms of moles, falling within the following ranges:

| | |
|---|---|
| $SiO_2/Al_2O_3$ or $B_2O_3$ | 20-1000 |
| $OH^-/SiO_2$ | 01-0.8 |
| $H_2O/OH^-$ | 20-100 |
| R/(R+Z) | 0.1-0.8 |
| K/K+Na | 0.2-1.0 |

wherein R is the 2-(hydroxylakyl)trialkylammonium cation and Z is alkali metal ion.

6. A method for preparing the synthetic porous crystalline material of Claim 2, which comprises preparing a mixture containing sources of alkali metal ions, an oxide of aluminum, an oxide of silicon, a 2-(hydroxyalkyl)trialkylammonium cation and water having a composition, in terms of moles, falling with the following ranges:

| | |
|---|---|
| $SiO_2/Al_2O_3$ | 20 - 1000 |
| $OH^-/SiO_2$ | 0.1 - 0.8 |
| $H_2O/OH^-$ | 20 - 100 |
| R/(R+Z) | 0.1 - 0.8 |
| K/(K+Na) | 0.2 - 1.0 |

wherein R is the 2-(hydroxyalkyl)trialkylammonium cation, Z is the alkali metal ion, and said $Al_2O_3$ is provided by a source of limited solubility in said reaction mixture such that said source exhibits a Degree of Solubility of less than about 20 weight percent in water solution and less than about 90 weight percent in caustic solution, and maintaining said reaction mixture under sufficient conditions until said crystalline material is formed.

7. The method of Claim 6, wherein said mixture has a composition, in terms of moles, falling with the following ranges:

| | |
|---|---|
| $SiO_2/Al_2O_3$ | 20 - 250 |
| $OH^-/SiO_2$ | 0.2 - 0.6 |
| $H_2O/OH^-$ | 20 - 60 |
| R/(R+Z) | 0.2 - 0.6 |
| K/(K+Na) | 0.2 - 0.7 |

8. The method of any one of claims 4 to 7 wherein said mixture further comprises a sufficient amount of seed crystals.

9. The method of any one of Claims 4 to 8 wherein said sufficient conditions comprise a temperature of from 60°C to 350°C and a pH of from 7 to 14.0

10. A process for effecting catalytic conversion of an organic charge which comprises contacting said charge under catalytic conversion conditions with a catalyst comprising a synthetic porous crystalline metallosilicate zeolite material of Claim 1.